(19) Europäisches Patentamt
European Patent Office
Office européen des brevets

(11) **EP 2 363 168 B1**

(12) **EUROPÄISCHE PATENTSCHRIFT**

(45) Veröffentlichungstag und Bekanntmachung des Hinweises auf die Patenterteilung:
**01.07.2015 Patentblatt 2015/27**

(51) Int Cl.:
***A61N 2/04*** *(2006.01)*      ***A61N 2/02*** *(2006.01)*

(21) Anmeldenummer: **11001448.7**

(22) Anmeldetag: **22.02.2011**

(54) **Vorrichtung zur Steuerung eines Frequenzgenerators zur Erzeugung von elektromagnetischen Feldern**

Device for controlling a frequency generator for generating electromagnetic fields

Dispositif de commande d'un générateur de fréquence pour la production de champs électromagnétiques

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **01.03.2010 DE 102010009743**

(43) Veröffentlichungstag der Anmeldung:
**07.09.2011 Patentblatt 2011/36**

(73) Patentinhaber:
• **Mazac, Karel**
 **86316 Friedberg (DE)**
• **Schuller, Paul**
 **86316 Friedberg (DE)**

(72) Erfinder: **Mazak, Karel Prof. Dr.**
 **86316 Friedberg (DE)**

(74) Vertreter: **Wiedemann, Markus**
 **Patentanwalt**
 **Ludwigstrasse 1**
 **86150 Augsburg (DE)**

(56) Entgegenhaltungen:
 **EP-A1- 1 894 600        WO-A2-02/35985**
 **WO-A2-02/085449      DE-A1- 19 708 542**
 **US-A- 5 450 859         US-A- 5 752 911**
 **US-A1- 2003 028 071   US-B1- 6 461 289**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft eine Vorrichtung und ein Verfahren zur Steuerung eines Frequenzgenerators, und bezieht sich insbesondere auf eine Vorrichtung und ein Verfahren zur Steuerung eines Frequenzgenerators zur Erzeugung von elektromagnetischen Wechselfeldern in einem vorbestimmten Frequenzbereich.

Ein solches Verfahren bzw. eine solche Vorrichtung ist aus US 2003/028071 A1 bekannt.

Es ist bekannt, dass stationäre, d.h. zeitlich konstante, Magnetfelder in der Umgebung von beispielsweise Permanentmagneten oder von mit Gleichstrom durch-flossenen Leitern entstehen.

Durch elektromagnetische Induktion sind unter Verwendung periodischer oder auch aperiodischer Steuerfunktionen zudem Impulspakete erzeugbar. Ferner sind Kombinationen aus stationären Magnetfeldern und Impulspaketen darstellbar.

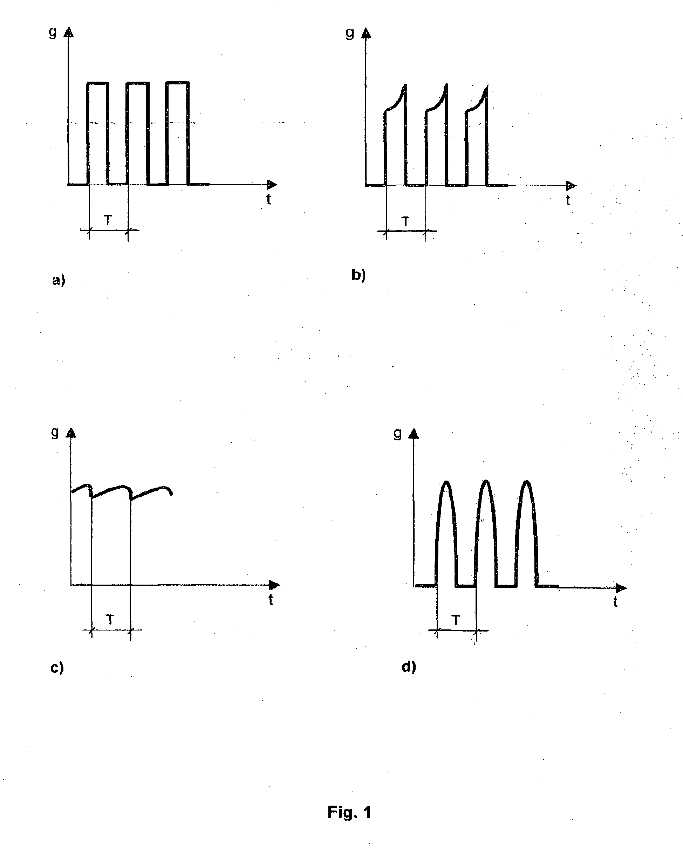

In Verbindung mit Anwendungen elektromagnetischer Felder wird im Stand der Technik über Impulsverfahren berichtet, die sich in Impulsform, Frequenz und/oder Intensität des Magnetfeldes unterscheiden. Impulsfunktionen sind solche, bei denen sich das Vorzeichen nicht ändert - die Amplitude verändert sich entweder nur im positiven oder nur im negativen Bereich entlang der Zeitachse. Die Frequenzbereiche werden als nieder- bis höherfrequent von ca. 1 Hz bis maximal 100 kHz reichend angegeben. Um beliebige Impulsformen erreichen zu können, werden die einzelnen Impulse analog zu industriellen Anwendungen aus einer Reihe von höherfrequenten Pulsen gebildet. Bei diesen Verfahren wird sowohl die Frequenzals auch die Pulsweitenmodulation angewendet. Einige Beispiele von impulsfunktionen für eine beliebige zeitabhängige physikalische Größe g, die überwiegend als Hüllkurve mehrerer Pulse entstehen, sind in den FIG. 1a, 1b, 1c und 1 d gezeigt, worin T die Periode der jeweiligen Impulsfunktion darstellt.

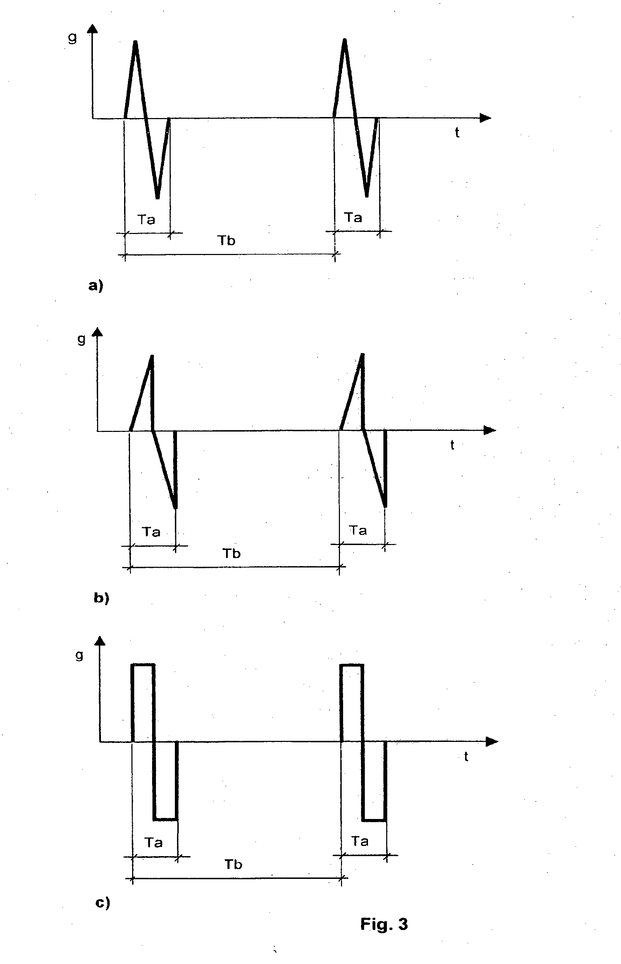

Des Weiteren sind Verfahren bekannt, bei den periodische Schwingfunktionen in Form von symmetrischen oder asymmetrischen Sägezähnen als Einzelwelle mit einer Periode von z.B. 10 ms (Ta, entsprechend 100 Hz) mit einer Wiederholungsrate von 100 ms (Tb, entsprechend 10 Hz) angewendet werden. Beispiele solcher periodischer Schwingfunktionen sind in den FIG. 3a, 3b und 3c gezeigt.

Ferner ist die Anwendung von Sinusfunktionen oder semi-Sinusverläufen als den wichtigsten periodischen Funktionen überhaupt bekannt. Allerdings erwies sich deren Anwendung für therapeutische Zwecke weder unmittelbar, noch mittelbar, noch nachhaltig als wirksam.

Der Erfindung liegt die Aufgabe zugrunde, eine Vorrichtung und ein Verfahren zur Steuerung eines Frequenzgenerators zur Erzeugung elektromagnetischer Felder zu schaffen, mit welchen eine Abstimmung und Steuerung für ein geeignetes Verhältnis der die resultierenden elektromagnetischen Felder bestimmenden Größen ermöglicht wird.

Diese Aufgabe wird durch eine Vorrichtung zur Steuerung eines Frequenzgenerators für die Erzeugung eines elektromagnetischen Felds gemäß den Merkmalen des Patentanspruchs 1 und durch ein Verfahren zur Steuerung eines Frequenzgenerators für die Erzeugung eines elektromagnetischen Felds gemäß den Merkmalen des Patentanspruchs 13 gelöst.

Vorteilhafte Weiterbildungen der Erfindung sind Gegenstand der beigefügten Unteransprüche.

Die der Erfindung zugrunde liegende Idee beruht auf einer Kombination höherer Frequenzen, bevorzugt Frequenzen im kHz-Bereich, mit einer neuartigen überlagerten Modulation im niederfrequenten Bereich in einem Steuerungssystem und/oder einem Steuerungsverfahren für eine Generatorsteuerung zur Erzeugung von Magnet-Wechselfeldern derart, dass ausschließlich Schwing- oder Wechselfunktionen (und damit keine Impuls- bzw. Pulsfunktionen) mit einem rechteckförmigen Spannungsverlauf und trapezförmigen Stromverlauf zur Anwendung kommen.

Gemäß der zugrunde liegenden Idee lassen sich diese Verläufe aus einem Frequenzbereich von beispielsweise 6 bis 60 kHz durch frei wählbare Zeitabschnitte kontinuierlich übergeordnet modulieren, wobei die übergeordnete Modulation vorzugsweise in einem Bereich zwischen 0,1 bis 10 Hz liegt. Technisch möglich wäre auch ein Bereich zwischen 0 (kein Wechselfeld) und 50 Hz, sofern eine Kontinuität bei Frequenzen höher 10 Hz darstellbar ist. Praktisch betrachtet ergibt sich dadurch bei einer Abschnittszeit von einer Sekunde eine übergeordnete Modulation von 0,5 Hz, bei einer Abschnittszeit von 100 ms eine übergeordnete Modulation von 5 Hz, und so weiter. Die Frequenzwerte werden entsprechend dem Takt des Mikroprozessors geändert, beispielsweise tausendmal pro Sekunde. Analog ist diese Vorgehensweise auch auf andere Betriebsarten bzw. alle bereitgestellten Betriebsarten anwendbar.

Für die Erzeugung eines elektromagnetischen Felds werden somit ausschließlich periodische Schwingfunktionen angewendet. Mittels viereckigen Spannungspulsen werden symmetrische trapezförmige Strompulse mit frei einstellbarem Verhältnis zwischen einem dynamischen und einem statischen Anteil erzeugt. Für einen Prozessablauf kann zwischen Betriebsarten eines Konstantmodus, eines Frequenzmodus, eines Pulsweitenmodus und eines den Frequenzmodus und den Pulsweitenmodus kombinierenden Magnetflussmodus gewählt werden.

Somit wird die vorstehende Aufgabe gelöst durch eine Vorrichtung zur Steuerung eines Frequenzgenerators für die Erzeugung eines elektromagnetischen Felds, wobei die Vorrichtung dazu angeordnet ist, am Ausgang des Frequenzgenerators periodische Schwing- oder Wechselfunktionen beliebiger Formen in Übereinstimmung mit einer ersten Betriebsart zu erzeugen und diese in Übereinstimmung mit zumindest einer weiteren Betriebsart kontinuierlich zu verändern.

Bevorzugt bewirken die kontinuierlichen Veränderungen eine Hintergrundmodulation im Bereich von 0,1 bis 10 Hz. Dieser niederfrequente Bereich kann vorteilhaft die Wirkungen der resultierenden Änderungen des elektromagnetischen Felds auf ein Zielobjekt verstärken.

Vorteilhaft ist die zumindest eine weitere Betriebsart eine zweite Betriebsart, bei der eine Frequenz kontinuierlich verändert wird, während eine Pulsweite prozentual konstant bleibt. Zusätzlich zu bekannten allgemeinen Vorteilen einer Frequenzmodulation lässt sich eine kontinuierliche Frequenzveränderung günstig und zweckmäßig in ein robustes Steuerungssystem integrieren.

Alternativ ist die zumindest eine weitere Betriebsart eine dritte Betriebsart, bei der eine Pulsweite kontinuierlich verändert wird, während eine Frequenz konstant bleibt. Bei der Realisierung der kontinuierlichen Pulsweitenveränderung beispielsweise mittels bevorzugter Weise MOSFETs oder IGBTs können diese vorteilhaft im verlustarmen Schaltbetrieb arbeiten. Weiter vorteilhaft ist die darzustellende Information anstelle in einer binären Aussage in einem kontinuierlichen Pulsbreitenverhältnis enthalten.

Weiter alternativ ist die zumindest eine weitere Betriebsart eine Kombination einer zweiten und einer dritten Betriebsart, bei der sowohl eine Frequenz als auch eine Pulsweite kontinuierlich verändert wird. Diese Betriebsart verbindet besonders vorteilhaft die Vorteile der kontinuierlichen Pulsweitenveränderung und der kontinuierlichen Frequenzveränderung.

Hierbei werden bevorzugt sowohl die Frequenz als auch die Pulsweite kontinuierlich derart verändert, dass der magnetische Fluss konstant bleibt. Vorteile ergeben sich dadurch, dass bei Anwendungen, bei welchen ein konstanter magnetischer Fluss wesentlich ist, dieser durch geeignete Vorgaben gewährleistet werden kann. Vorteilhaft ist das zeitliche Verhältnis zwischen einem dynamischen und einem statischen Verlauf eines magnetischen Felds frei einstellbar. Aufgrund der unterschiedlichen Wirkungen dynamischer und statischer magnetischer Felder kann hierdurch eine gewünschte Gesamtwirkung fein abgestimmt werden.

Auch vorteilhaft ist es, wenn Grenzwerte für alle Betriebsarten frei einstellbar sind. Dies stellt sicher, dass anwendungsbezogen gegebenenfalls vorhandene Grenzwerte sicher eingehalten werden können.

Ferner vorteilhaft ist es, wenn für die einzelnen Betriebsarten benötigte Zeitkonstanten sämtlich frei einstellbar sind. Dies verbessert die Steuerbarkeit des Frequenzgenerators und damit des zu erzeugenden elektromagnetischen Felds.

Bevorzugt weisen die Betriebsarten Signalverläufe mit variabler Periodenzahl auf. Dies verbessert ebenfalls vorteilhaft die Steuerbarkeit des zu erzeugenden elektromagnetischen Felds.

Alternativ oder gleichzeitig bevorzugt weisen die Betriebsarten Signalverläufe mit konstanter Periodenzahl auf. Dies verbessert ebenfalls vorteilhaft die Steuerbarkeit des zu erzeugenden elektromagnetischen Felds.

Bevorzugt beinhaltet die Vorrichtung dazu einen Generator, der zumindest eine Induktionsspule zur Erzeugung des elektromagnetischen Felds mit Wechselstrom in Form von periodischen Schwingfunktionen beaufschlagt. Ein solcher Aufbau erlaubt vorteilhaft eine effiziente und kostengünstige Darstellung einer Vorrichtung sowie einen gut beherrschbaren Verfahrensablauf.

Weiter bevorzugt weist der Generator eine frei programmierbare Steuerung mit einer Bedieneroberfläche auf. Dadurch werden vorteilhaft die Bedienung einer Vorrichtung und eine Abstimmung im Hinblick auf eine zu erzielende Wirkung erleichtert

In diesem Fall kann vorteilhaft die frei programmierbare Steuerung auf Mikroprozessorbasis aufgebaut sein. Ein geeignet wählbarer Mikroprozessor erlaubt insbesondere einen kostengünstigen Aufbau bei gleichzeitig beherrschbarer Programmierbarkeit, und somit eine einfache Aktualisierung und/oder Erweiterung der Funktionalität.

Die vorstehende Aufgabe wird ebenfalls gelöst durch ein Verfahren zur Steuerung eines Frequenzgenerators für die Erzeugung eines elektromagnetischen Felds, bei dem am Ausgang des Frequenzgenerators periodische Schwing- oder Wechselfunktionen beliebiger Formen in Übereinstimmung mit einer ersten Betriebsart erzeugt und diese in Übereinstimmung mit zumindest einer weiteren Betriebsart kontinuierlich verändert werden.

Weitere Verfahrensschritte können einzeln oder in Kombination die vorstehend beschriebenen vorteilhaften und/oder bevorzugten Ausgestaltungen realisieren.

Die Erfindung wird nachstehend anhand von Ausführungsbeispielen unter Bezugnahme auf die Zeichnung näher beschrieben. Es zeigen:

**Fig. 1a bis 1d** einige Beispiele von Impulsfunktionen für eine beliebige zeitabhängige physikalische Größe g, die überwiegend als Hüllkurve mehrerer Pulse entstehen, worin T die Periode der jeweiligen Impulsfunktion darstellt;

**Fig. 2a** eine Gesamtanordnung der Vorrichtung gemäß einer Ausführungsform, bestehend aus einem mittelfrequenten Generator **a**, Verbindungsleitungen **b**, und einer oder mehreren Induktionsspulen c, und **Fig. 2b** eine mit einem Spannungszwischenkreis arbeitende und als H-Brücke verschaltete Endstufe des Generators a;

**Fig. 3a bis 3c** einige Beispiele periodischer Schwingfunktionen in Form von symmetrischen oder asymmetrischen Sägezähnen als Einzelwelle mit einer Periode und einer Wiederholungsrate;

**Fig. 4a** rechteckförmige Spannungspulse mit einer Pulsweite von 100 %, und **Fig. 4b** rechteckförmige Spannungspulse mit einer Pulsweite von 50 %;

**Fig. 5a und Fig. 5b** trapezförmige Funktionsverläufe ohne Pulsunterbrechung, die bei einer Begrenzung der maximal zulässigen Pulsweite in den **Fig. 4a** und **4b** nach oben bei sowohl den Größen Strom als auch magnetischem Fluss entstehen;

**Fig. 6** einen symmetrischen Spannungsverlauf, der ein periodisches bzw. aperiodisches Durchlaufen eines beliebigen, frei wählbaren Frequenzbereichs bei einer Frequenzmodulation mit gleichbleibender Pulsweite in einer zweiten Betriebsart darstellt;

**Fig. 7** einen symmetrischen Spannungsverlauf, der ein periodisches bzw. aperiodisches Durchlaufen eines beliebigen Pulsweitenbereichs bei einer Pulsweitenmodulation mit gleichbleibender Frequenz in einer dritten Betriebsart darstellt;

**Fig. 8** einen asymmetrischen Spannungsverlauf, der ein periodisches bzw. aperiodisches Durchlaufen eines beliebigen, frei wählbaren Frequenzbereichs bei einer Frequenzmodulation mit gleichbleibender Pulsweite in der zweiten Betriebsart darstellt; und

**Fig. 9** einen asymmetrischen Spannungsverlauf, der ein periodisches bzw. aperiodisches Durchlaufen eines beliebigen Pulsweitenbereichs bei einer Pulsweitenmodulation mit gleichbleibender Frequenz in der dritten Betriebsart darstellt;

Bei beispielsweise einer EMF-Therapie handelt es sich um eine induktive, d. h. berührungs- bzw. kontaktlos erfolgende Energieübertragung. Für ihre Umsetzung ist ein System notwendig, das aus einem Frequenzgenerator und mindestens einer Induktionsspule besteht. Der Generator stellt an seinem Ausgang die notwendige Spannung mit einem bestimmten Frequenzbereich zur Verfügung. Wird an diesem Ausgang eine Induktionsspule angeschlossen, fließt durch diese ein Wechselstrom, der einen zeitabhängigen magnetischen Fluss zur Folge hat.

Wird im Weiteren eine Funktion als "Impuls" oder "Puls" bezeichnet, handelt es sich immer um einen Funktionsverlauf während einer Halbperiode. Danach folgt immer ein Puls mit umgekehrtem Vorzeichen. Bei einem Spannungsverlauf erfolgen die positiven und negativen Pulse mit einer Nullphase zwischen den beiden Halbwellen, bei einem Stromverlauf und/oder einem Feldverlauf ist diese Folge lückenlos. In diesem Fall bilden die beiden Halbwellen eine periodische Wechselfunktion.

Sowohl die Spannungsamplitude als auch der Frequenzbereich sind durch verfahrenstechnische Randbedingungen bestimmt und eng miteinander verknüpft. Physikalisch gesehen können tiefe Frequenzen kaum die im Gewebe notwendigen Potentialunterschiede entstehen lassen und damit auch während der relativ kurzen Expositionszeiten eine therapeutische Wirkung entfalten. Hohe Frequenzen dagegen begrenzen entweder die Größe der Induktionsspule oder die erreichbare Feldstärke, d.h. sie schränken den Behandlungsbereich stark ein. Der Ausgleich über höhere Spannungswerte ist aus Sicherheitsgründen unerwünscht. Darüber hinaus soll bei dieser Form der Therapie keine direkte Gewebeerwärmung stattfinden.

Die Begründung dazu liefern die physikalischen Feldeigenschaften. Es ist rein theoretisch nur schwer möglich und praktisch unmöglich, bei niedrigen Frequenzen und relativ schwachen Feldern etwa biologisch-chemische oder physiologische Wirkungen zu erzielen, da allgemein eine Verstärkung oder ein Hervorrufen einer induktiven Wirkung durch hohe Frequenzen erzielt wird. Dagegen können ein Körper und/oder Zellen gut auf Frequenzen im niedrigen Bereich, d. h. langsame Modulationen, die deren Ansprechbereitschaft erhöhen, reagieren.

Zahlreiche Versuche haben gezeigt, dass keine ganz bestimmte Frequenz oder kein ganz bestimmter Frequenzbereich notwendig sind, um nachhaltige therapeutische Ergebnisse erzielen zu können. Vielmehr ist es wichtig , dass alle Systemkomponenten richtig auf einander abgestimmt sind, d.h. der Frequenzbereich, die Spannungsamplitude, die Impulsbreite und die Induktivitäten der gesamten Induktorpalette müssen in einem ganz bestimmten Verhältnis zueinander stehen. Sollte aus Behandlungsgründen der Frequenzbereich weiter erweitert werden, wäre zusätzlich eine Amplitudenmodulation der Ausgangsspannung notwendig.

Die so oft angesprochene Wirkung einer Gewebeerwärmung durch elektromagnetische Induktion ist in dem erfindungsgemäß angegebenen Frequenzbereich bei Induktionen unterhalb von 10 mT und Expositionszeiten von maximal 30 Minuten so gut wie ausgeschlossen. Diese Feststellung basiert auf langfristigen und umfangreichen Versuchen mit ferromagnetischen und nicht ferromagnetischen Nanopartikeln in unterschiedlichsten Konzentrationen mit Frequenzen von 8 kHz bis 50 kHz und magnetischen Feldstärken bis zu 500 mT.

Der für die Wirkung notwendige magnetische Fluss $\phi$ ist direkt proportional zu dem elektrischen Strom I, der ihn verursacht. Die Proportionalkonstante heißt Induktivität L und stellt die charakteristische Größe eines Induktors dar:

$$\Phi = L \times I$$

Die vorstehende Gleichung besagt gleichzeitig, dass der Verlauf des magnetischen Flusses in linearen Systemen dem Stromverlauf treu folgt. Steigt der Strom linear, steigt auch der Fluss linear, ist der Strom konstant, nimmt auch der Fluss einen konstanten Wert an.

Da es sich in diesen Fällen um lineare Systeme und zeitabhängige Verläufe, d.h. um Wechselströme handelt, kann die Gleichung nach der Zeit differenziert werden:

$$d\Phi / dt = L \times di / dt$$

wobei die induzierte Spannung

$$u = - d\Phi / dt$$

ist. Das Zielobjekt - im Beispiel ein (Körper)Gewebe - ist eine nicht homogene Materie, und die Magnetfeldstärke ist distanzabhängig. Deshalb entstehen in diesem Zielobjekt Potentialunterschiede, die ein elektrisches Feld zur Folge haben:

$$E = u2 - u1 = - grad\ u$$

Dadurch wirkt auf elektrisch geladene Partikel - im Beispiel Ionen - eine Kraft:

$$F = E \times q$$

Die Ionenbeweglichkeit wird erhöht und in eine bestimmte Richtung gelenkt. Dadurch können mehr Ionen in die Zelle transportiert werden, und die negative Spannung der Zelle kann schnell auf den für den "Normalbetrieb" notwendigen Wert von etwa (-70 bis -90 mV) ansteigen. Dieser Vorgang kann analog zur Aufladung eines Kondensators C mit der Ladung:

$$Q = I \times t$$

und der resultierenden Spannung:

$$U = Q / C$$

dargestellt werden. Vor diesem Hintergrund sollen nun ein Verfahren und eine Vorrichtung auf der Basis elektromagnetischer Felder bereitgestellt werden, welche eine höhere Effektivität und eine breitere Anwendbarkeit der elektromagnetischen Felder, beispielsweise bei therapeutischen Effekten bzw. Wirkungen gewährleisten.

Als besonders vorteilhaft hat sich eine senkrecht zum Objekt verlaufende Richtung der Feldlinien gezeigt. Die Magnetfeldstärke - die Induktion - des durch die Spule produzierten Magnetfeldes wird mittels einer Frequenz- und/oder einer Pulsweitenmodulation gesteuert.

Nachstehend wird hierbei eine Steuerung mittels einer Frequenzmodulation als eine zweite Betriebsart bezeichnet, eine Steuerung mittels einer Pulsweitenmodulation als eine dritte Betriebsart bezeichnet, und eine Steuerung mittels einer Kombination aus einer Frequenzmodulation und einer Pulsweitenmodulation als eine weitere Betriebsart bezeichnet.

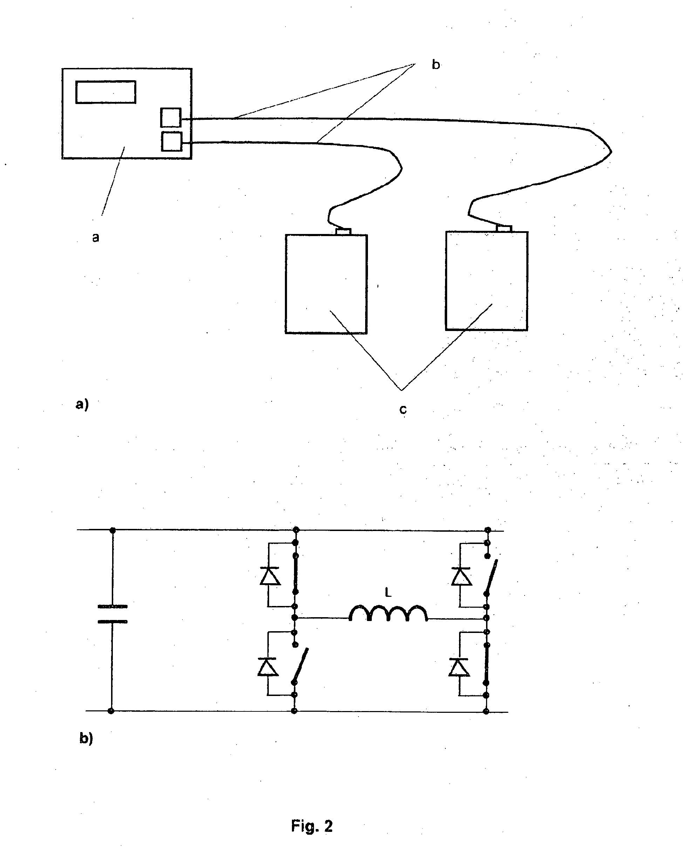

Mit Hilfe von theoretischen Betrachtungen, Literaturvergleichen und praktischen Erfahrungen wurde etwa ein neues, auf dem Gebiet der Magnetfeldtherapie anwendbares Verfahren entwickelt, bei welchem die hierin beschriebene Vorrichtung und das hierin beschriebene Verfahren anwendbar sind. Das Verfahren basiert auf einer neuartigen Pulsform und einer Reihe verschiedener Verfahrensmodi oder Betriebsarten. Das gesamte System besteht gemäß **FIG. 2a** aus einem mittelfrequenten Generator **a,** Verbindungsleitungen **b,** und einer oder mehreren Induktionsspulen **c** (Induktor, Antenne, etc.), die anwendungsabhängig verschiedene Formen wie beispielsweise kreisförmig, oval, eckig, flach und dergleichen aufweisen können. Eine Endstufe des Generators arbeitet mit einem Spannungszwischenkreis und ist wie in **Fig. 2b** gezeigt ausschließlich als H-Brücke geschaltet. Die Leistungselemente, beispielsweise MOSFETs oder IGBTs, sind in der Figur als Schalter mit zugehörigen Freilaufdioden dargestellt. Nur diese Schaltung ermöglicht die Ausnutzung der in der Induktionsspule gespeicherten Energie für die Erzeugung von trapezförmigen Pulsen ohne zusätzliche Frequenz- oder Pulsweitenmodulation. Die Generatorsteuerung ist so aufgebaut, dass die einzelnen weiter beschriebenen Verfahrensmodi frei programmierbar und speicherbar sind.

Der wirksamste Frequenzbereich liegt zwischen 5 kHz und 50 kHz.

Die für verschiedene Behandlungen notwendige Palette von Induktionsspulen wird mit quasi gleichen Eigenschaften ausgelegt, so dass bei keiner Einstellung weder die zulässigen Ströme noch die angegebenen Feldstärken überschritten werden können. In besonderen Fällen kann der anwendbare Frequenzbereich eingeschränkt werden.

Der Effektivwert der am Induktor anliegenden Spannung reicht von einigen Volt bis etwa 50 V. Die elektrische Ausführung entspricht somit einer Ausführung mit Sicherheits- oder Schutzkleinspannung (SELV; safe extra low voltage). Lediglich für den veterinären Anwendungsbereich kann bei großflächigen oder großräumigen Spulen der 50 V - Grenzwert bedarfsweise überschritten werden.

Es werden ausschließlich periodische Schwingfunktionen angewendet, die sowohl positive als auch negative Werte annehmen, ohne dass diese in einem definierten Verhältnis zueinander stehen müssen. Eine periodische Funktion erfüllt die Gleichung:

$$f\,(t + T) = f\,(t)$$

Im Grenzfall gehen die Schwingfunktionen in Wechselfunktionen über, wobei die positiven und negativen, durch die Spannungskurve begrenzten Flächen innerhalb einer Periode identisch sind.

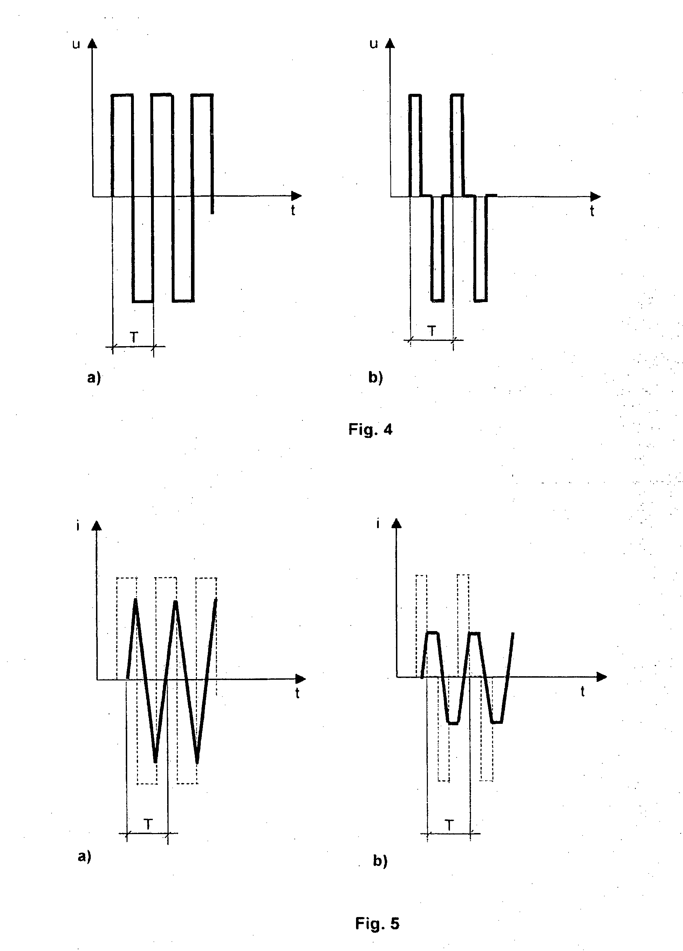

Sowohl die positiven als auch die negativen Spannungspulse sind rechteckig. In **FIG 4a** ist die Pulsweite PW mit 100 %, in **FIG. 4b** mit 50 % dargestellt. Die Steuersignale für den Strom- und damit auch für den Flussverlauf werden so erzeugt, dass es bei beiden unabhängig von der jeweiligen Pulsweite zu keiner Unterbrechung kommt. Wird die maximal zulässige Pulsweite nach oben begrenzt - z.B. mit 75 % - entstehen bei beiden Größen - Strom und Fluss - unterbrechungsfreie trapezförmige Funktionsverläufe ohne Pulsunterbrechung, wie in den **FIG. 5 a** und **5b** dargestellt.

Die Abstimmung zwischen den einzelnen Systemkomponenten erfolgt so, dass ein (in der Figur darstellerisch) waagrechter Strom-/Fluss-Verlauf durch die in der Induktionsspule gespeicherte Energie aufrecht erhalten wird. Die Zeit und damit auch die notwendige Energie für diesen waagrechten Verlauf ergeben sich zu:

$$t = T/2 - PW$$

wobei PW die Spannungs-Puls-Weite darstellt.

Je nach Systemverlusten und der Menge der in der Induktionsspule gespeicherten Energie kann der Stromwert während dieser Phase leicht abfallend sein; dies kann jedoch keinen Einfluss auf das Ergebnis haben. Solange

$$di/dt \rightarrow 0$$

erfüllt ist, kann von einem "quasi waagrechten" oder "quasi trapezförmigen" Funktionsverlauf gesprochen werden. Während dieses Verlaufs verhält sich das System als Permanentmagnet, dessen Polarität mit wechselnder Polarität der Pulsfunktion wechselt.

Reicht die im Induktor gespeicherte Energie für die Aufrechterhaltung des Stromes nicht aus, muss eine der Systemgrößen - die Frequenz, die Spannungs-Puls-Weite PW oder die Induktivität - entsprechend angepasst werden, um diesem Effekt entgegen zu wirken. Eine zusätzliche höherfrequente Frequenzmodulation ist für die Entstehung dieser Pulsformen nicht notwendig.

Zusammengefasst kommt es somit bei trapezförmigen Verläufen während einer Halbperiode abwechselnd zu dynamischen und statischen Wirkungen, d. h. einer wechselnden Wirkung von Wechselfeldern und Permanentmagneten.

Sollte aus beispielsweise therapeutischen Gründen der stationäre Feldanteil erhöht werden, werden die Steuerimpulse so eingestellt, dass sich die positiven und die negativen Halbwellen bei gleicher Amplitude in der Pulsweite voneinander unterscheiden. Das Ergebnis gleicht einer Vormagnetisierung entweder in positiver oder in negativer Richtung.

Der Basismodus oder Konstantmodus bzw. die erste Betriebsart für die Anwendung beinhaltet eine konstante Frequenz f und eine konstante Pulsweite PW. Die Einrichtung arbeitet damit in einem so genannten

## t_mode bei f = konst. und PW = konst.

Um die Wirkung der erzeugten elektromagnetischen Felder besser an einen Anwendungsfall anpassen zu können (beispielsweise um verschiedene Gewebestrukturen intensiver ansprechen zu können), sind zumindest drei weitere Modi oder Betriebsarten der Pulssteuerung vorgesehen.

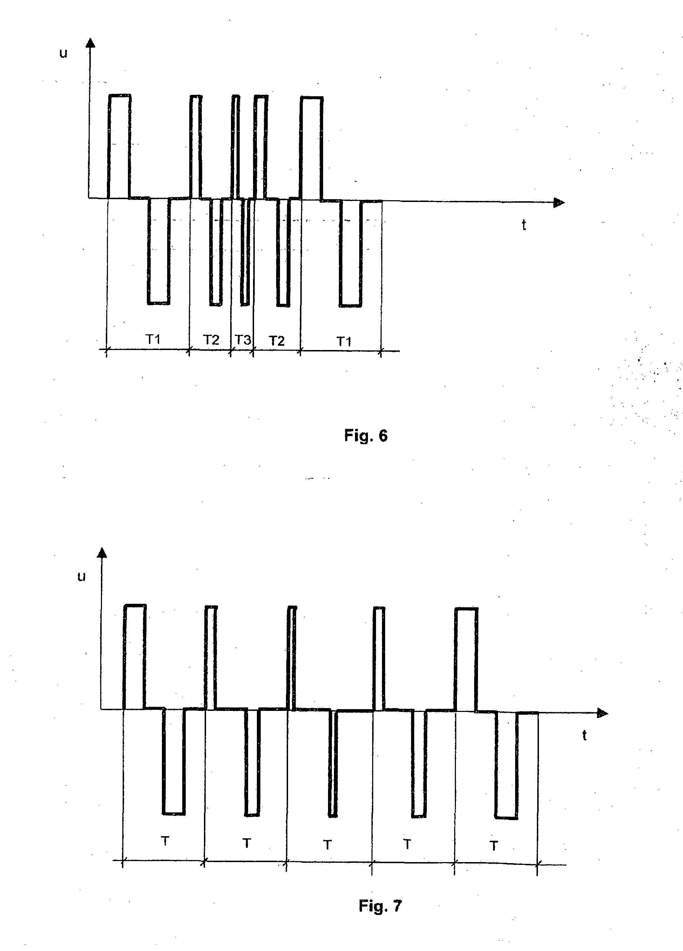

Ein zweiter Modus oder Frequenzmodus bzw. eine zweite Betriebsart ermöglicht über entsprechende Steuerungsvorgaben ein periodisches bzw. aperiodisches Durchlaufen eines beliebigen frei wählbaren Frequenzbereichs, wobei die Frequenz über entsprechende frei wählbare Zeitkonstanten erhöht oder abgesenkt wird. Die Pulsweite bleibt dabei prozentual konstant. Dies entspricht einer in **Fig. 6** gezeigten Frequenzmodulation:

## f_mode bei PW = konst.

In **FIG.6** wurde die Pulsweite PW = 50 % gewählt. Die einzelnen Pulsweiten sind stellvertretend für ein Paket von gleichen Pulsen. Entspricht der erste positive Puls z.B. einer Frequenz von 10 kHz und wird die Frequenz entsprechend der Zykluszeit einer Mikroprozessorsteuerung von 1 ms stufenweise erhöht, ergeben sich nebeneinander 10 Pulse mit der gleichen Pulsweite - sowohl in positiver als auch in negativer Richtung. Die Schrittgröße für die Erhöhung bzw. Absenkung der Frequenz ergibt sich automatisch aus den vorgewählten Grenzwerten und aus der Zeit für einen Frequenzzyklus.

Ein dritter Modus oder Pulsweitenmodus bzw. eine dritte Betriebsart ermöglicht über entsprechende Steuerungsvorgaben ein periodisches bzw. aperiodisches Durchlaufen eines beliebigen Pulsweitenbereiches bei konstanter Frequenz. Dies entspricht einer in **Fig. 7** gezeigten Pulsweitenmodulation:

## PW_mode bei f = konst.

Die Vorgehensweise bei diesem Modus ergibt sich analog zu dem zweiten Modus f_mode.

Beide Modi (Frequenzmodus und Pulsweitenmodus) bzw. die zweite und die dritte Betriebsart haben gleichzeitig eine Stromänderung und damit auch eine Flussänderung zur Folge. Beide Größen können kontinuierlich, d.h. rampenförmig, oder sprunghaft zwischen zwei frei wählbaren Grenzwerten verändert werden.

Das erfindungsgemäße Verfahren sieht vor, dass alle Änderungen symmetrisch oder asymmetrisch vorgenommen werden können. Alternativ kann der Ablauf bei beiden Modi bzw. der zweiten und der dritten Betriebsart nur in einer Richtung erfolgen, z.B. in einem Frequenzzyklus von 10 kHz bis 30 kHz. Nach Erreichen der 30 kHz Grenze erfolgt automatisch ein Sprung zurück auf 10 kHz, wonach die Frequenz wieder auf 30 kHz ansteigt. Der Frequenzzyklus kann mit variabler Pulszahl und gleicher Zeit für alle Frequenzen - d.h. mit steigender Frequenz mit höherer Pulszahl - oder mit gleicher Schrittgröße - z.B. 1 kHz - und gleicher Pulszahl - z.B. 10 - für alle Frequenzen erfolgen. Die zweite Einstellung ergibt dann einen Frequenzzyklus von 13,3 s. Sowohl die Schrittgröße als auch die Pulszahl sind wieder frei wählbare Größen.

Analog gelten diese Ausführungen auch für den PW-Zyklus.

Somit zielt diese Vorgehensweise auf eine Frequenz- bzw. "Impuls"-Bandwirkung, die sowohl applikations- als auch objektabhängig gewählt werden kann. Werden beispielsweise einstellbare Zeitabschnitte als feste Werte gewählt, müssen dem entsprechend die "Bänder" manuell vorgewählt werden. Sind die Zeitabschnitte ebenfalls eine Funktion der Zeit, werden während der Anwendung beliebig viele "Bänder" durchlaufen, wodurch die Wahrscheinlichkeit einer positiven Wirkung erhöht wird.

In **FIG. 6** und **FIG. 7** sind symmetrische Spannungsverläufe dargestellt. Die entsprechenden Strom- und Flussverläufe ergeben sich dann analog zu **FIG. 5b**.

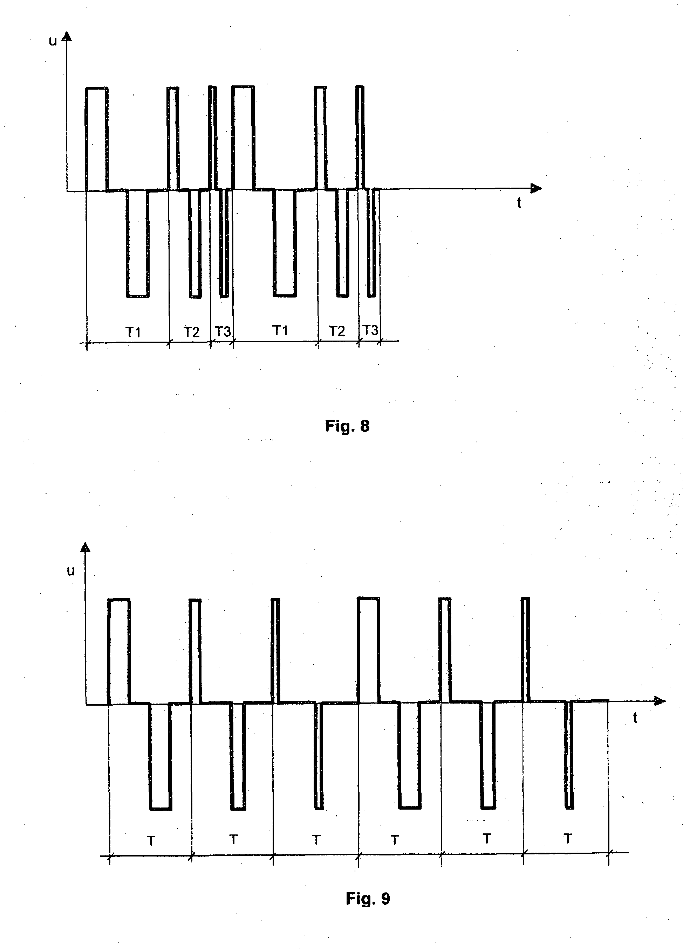

In **FIG. 8** und **FIG. 9** sind asymmetrische Spannungsverläufe dargestellt. Die entsprechenden Strom- und Flussverläufe ergeben sich erneut ebenfalls analog zu **FIG. 5b**.

Der weitere Modus bzw. die weitere Betriebsart beinhaltet in Form eines Magnetflussmodus die Kombination der beiden vorstehend erwähnten Modi (Frequenzmodus und Pulsweitenmodus) mit dem Ziel, den magnetischen Fluss konstant zu halten:

## Φ_mode bei f variabel und PW variabel

Mit steigender Frequenz und konstanter Pulsweite nimmt der Strom durch die Induktionsspule und damit auch der Magnetfluss ab. Um den Magnetfluss konstant zu halten, wird daher bei steigender Frequenz die Pulsbreite so erhöht, dass der durch die Induktionsspule fließende Strom konstant bleibt.

Alle Vorgaben können über beliebige, frei wählbare Zeit- oder Pulszahl-Konstanten entweder manuell (mittels beispielsweise einer Tastatur an einem Gerät) oder automatisiert (mittels beispielsweise einem Personal Computer und/oder einer übergeordneten Steuerung) eingestellt werden.

Wie vorstehend beschrieben wurde, beinhaltet eine Vorrichtung zur Steuerung eines Frequenzgenerators zur Erzeugung von elektromagnetischen Feldern einen Frequenzgenerator mit zumindest einer Induktionsspule zur Erzeugung eines elektromagnetischen Felds mit Wechselstrom in Form von periodischen Schwingfunktionen, und werden bei einem Verfahren zur Steuerung eines Frequenzgenerators zur Erzeugung von elektromagnetischen Feldern bei einem mittelfrequenten Generator ausschließlich periodische Schwingfunktionen mit trapezförmigem Stromverlauf erzeugt und angewendet.

Positive und negative Werte der Schwingfunktionen über die jeweilige Halbperiode stehen bevorzugt in keinem definierten Verhältnis zueinander. Die am Generatorausgang anstehende Spannung wird bevorzugt in Form von viereckigen Pulsen erzeugt. Der durch die Spule fließende Strom nimmt bevorzugt eine symmetrische Trapezform an. Das zeitliche Verhältnis zwischen dem dynamischen - abfallende und ansteigende Flanke - und dem statischen - parallel zur Zeitachse - Pulsverlauf ist frei einstellbar. Die trapezförmigen Strompulse werden bevorzugt als Einzelpulse ohne zusätzliche Frequenzmodulation erzeugt. Der frei einstellbare Frequenzbereich liegt vorzugsweise zwischen 5 und 50 kHz. Das System arbeitet im Frequenzmodus bei konstanter Pulsweite. Alternativ arbeitet das System im Pulsweitenmodus bei konstanter Frequenz. Die Frequenz und die Pulsweite können in gegenseitiger Abhängigkeit so verändert werden, dass der Magnetfluss konstant bleibt. Die Grenzwerte sind für alle Modi frei einstellbar. Alle für die einzelnen Modi benötigten Zeitkonstanten sind frei einstellbar. Die Signalverläufe verlaufen symmetrisch oder alternativ asymmetrisch. Die Abläufe können mit variabler Pulszahl erfolgen. Die Abläufe können alternativ mit konstanter Pulszahl erfolgen. Der Generator weist hierzu eine freiprogrammierbare Steuerung mit einer Bedieneroberfläche auf. Die freiprogrammierbare Steuerung ist dazu auf Mikroprozessorbasis aufgebaut. Die Induktionsspule ist dabei so ausgebildet, dass der magnetische Fluss senkrecht zu einer Oberfläche eines Zielobjekts erzeugt wird.

Im Hinblick auf gewerbliche Anwendbarkeit wurde mit Hilfe theoretischer Betrachtungen und praktischer Erfahrungen eine Vorrichtung zur Steuerung eines Frequenzgenerators dargestellt, welche auch zur Behandlung von Gewebe und/oder Zellen unter der Verwendung von elektromagnetischen Feldern nutzbar sind. Weiterhin sind beliebige Anwendungen der erfindungsmäßen Vorrichtung möglich.

**Patentansprüche**

1. Vorrichtung zur Steuerung eines Frequenzgenerators für die Erzeugung eines elektromagnetischen Felds, wobei sie ausgebildet ist, am Ausgang des Frequenzgenerators periodische und einen rechteckförmigen Spannungsverlauf sowie einen trapezförmigen Stromverlauf aufweisende Schwing- oder Wechselfunktionen beliebiger Formen in Übereinstimmung mit einer ersten Betriebsart zu erzeugen und diese in Übereinstimmung mit zumindest einer weiteren Betriebsart kontinuierlich zu verändern, **dadurch gekennzeichnet, dass** die zumindest eine weitere Betriebsart eine Kombinationsbetriebsart ist, bei der sowohl eine Frequenz als auch eine Pulsweite kontinuierlich derart verändert werden, dass der magnetische Fluss konstant bleibt.

2. Vorrichtung nach Anspruch 1, **dadurch gekennzeichnet, dass** sie ausgebildet ist, dass die kontinuierlichen Veränderungen eine Hintergrundmodulation im Bereich von 0,1 bis 10 Hz bewirken.

3. Vorrichtung nach Anspruch 1 oder 2, **dadurch gekennzeichnet, dass** die zumindest eine weitere Betriebsart eine zweite Betriebsart ist, bei der die Frequenz kontinuierlich verändert wird, während die Pulsweite prozentual konstant bleibt.

4. Vorrichtung nach Anspruch 1 oder 2, **dadurch gekennzeichnet, dass** die zumindest eine weitere Betriebsart eine dritte Betriebsart ist, bei der die Pulsweite kontinuierlich verändert wird, während die Frequenz konstant bleibt.

5. Vorrichtung nach einem der vorangehenden Ansprüche, **dadurch gekennzeichnet, dass** das zeitliche Verhältnis zwischen einem dynamischen und einem statischen Verlauf eines magnetischen Felds frei einstellbar ist.

6. Vorrichtung nach einem der vorangehenden Ansprüche, **dadurch gekennzeichnet, dass** Grenzwerte für alle Betriebsarten frei einstellbar sind.

7. Vorrichtung nach einem der vorangehenden Ansprüche, **dadurch gekennzeichnet, dass** für die einzelnen Betriebsarten benötigte Zeitkonstanten sämtlich frei einstellbar sind.

8. Vorrichtung nach einem der vorangehenden Ansprüche, **dadurch gekennzeichnet, dass** die Betriebsarten Signalverläufe mit variabler Periodenzahl aufweisen.

9. Vorrichtung nach einem der vorangehenden Ansprüche, **dadurch gekennzeichnet, dass** die Betriebsarten Signalverläufe mit konstanter Periodenzahl aufweisen.

10. Vorrichtung nach einem der vorangehenden Ansprüche, **gekennzeichnet, durch** einen Generator, der zumindest eine Induktionsspule zur Erzeugung des elektromagnetischen Felds mit Wechselstrom in Form von periodischen Schwingfunktionen beaufschlagt.

11. Vorrichtung nach Anspruch 10, **dadurch gekennzeichnet, dass** der Generator eine frei programmierbare Steuerung mit einer Bedieneroberfläche aufweist.

12. Vorrichtung nach Anspruch 11, **dadurch gekennzeichnet dass** die frei programmierbare Steuerung auf Mikroprozessorbasis aufgebaut ist.

13. Verfahren zur Steuerung eines Frequenzgenerators für die Erzeugung eines elektromagnetischen Felds, wobei am Ausgang des Frequenzgenerators periodische und einen rechteckförmigen Spannungsverlauf sowie einen trapezförmigen Stromverlauf aufweisende Schwing- oder Wechselfunktionen beliebiger Formen in Übereinstimmung mit einer ersten Betriebsart erzeugt und diese in Übereinstimmung mit zumindest einer weiteren Betriebsart kontinuierlich verändert werden, **dadurch gekennzeichnet, dass** die zumindest eine weitere Betriebsart eine Kombinationsbetriebsart ist, bei der sowohl eine Frequenz als auch eine Pulsweite kontinuierlich derart verändert werden, dass der magnetische Fluss konstant bleibt.

**Claims**

1. A device for controlling a frequency generator for generating an electromagnetic field, the frequency generator configured to generate periodic oscillating or alternating functions with any shape according to a first operating mode at an output of the frequency generator and to continuously vary the periodic oscillating and alternating functions according to at least one additional operating mode,
**characterized in that** the at least one additional operating mode is a combined operating mode in which a frequency and also a pulse width is continuously varied so that a magnetic flux remains constant.

2. The device according to claim 1, **characterized in that** the device is configured so that the continuous changes cause a background modulation in a range of 0.1 to 10 Hz.

3. The device according to claim 1 or 2, **characterized in that** the at least one additional operating mode is a second operating mode in which the frequency is continuously varied while the pulse width remains constant on a percent basis.

4. The device according to claim 1 or 2, **characterized in that** the at least one additional operating mode is a third operating mode in which the pulse width is continuously varied while the frequency remains constant.

5. The device according to one of the preceding claims, **characterized in that** a time ratio between a dynamic characteristic diagram and a static characteristic diagram of a magnetic field is freely adjustable.

6. The device according to one of the preceding claims, **characterized in that** threshold values for all operating modes are freely adjustable.

7. The device according to one of the preceding claims, **characterized in that** all time constants required for individual operating modes are freely adjustable.

8. The device according to one of the preceding claims, **characterized in that** the operating modes have characteristic signal diagrams with a variable number of periods.

9. The device according to one of the preceding claims, **characterized in that** the operating modes have characteristic signal diagrams with a constant number of periods.

10. The device according to one of the preceding claims, **characterized by** a generator which loads at least one induction coil with alternating current configured as periodic oscillation functions for generating an electromagnetic field.

11. The device according to claim 10, **characterized in that** the generator has a freely programmable control with a user interface.

12. The device according to claim 11, **characterized in that** the freely programmable control is based on microprocessors.

13. A method for controlling a frequency generator for generating an electromagnetic field,
    wherein periodic oscillating or alternating functions with a rectangular voltage diagram and a trapezoid current diagram and with any shape are generated according to a first operating mode at an output of the frequency generator and
    wherein the periodic oscillating and alternating functions are continuously varied according to at least one additional operating mode,
    **characterized in that** the at least one additional operating mode is a combined operating mode in which a frequency and also a pulse width are continuously varied so that a magnetic flux remains constant.

**Revendications**

1. Dispositif pour la commande d'un générateur de fréquence pour la production d'un champ électromagnétique, cependant qu'il est configuré pour produire, à la sortie du générateur de fréquence, des fonctions d'oscillation ou de changement périodiques et qui présentent une courbe de tension rectangulaire ainsi qu'un trajet de courant trapézoïdal de formes quelconques en conformité avec un premier mode opératoire et pour modifier celles-ci en continu en conformité avec au moins un autre mode opératoire, **caractérisé en ce que** l'autre mode opératoire qui existe au moins est un mode opératoire combiné pour lequel aussi bien une fréquence qu'une largeur d'impulsions sont modifiées en continu de telle manière que le flux magnétique reste constant.

2. Dispositif selon la revendication 1, **caractérisé en ce qu'**il est configuré tel que les modifications continues provoquent une modulation de fond dans la plage de 0,1 à 10 Hz.

3. Dispositif selon la revendication 1 ou 2, **caractérisé en ce que** l'autre mode opératoire qui existe au moins est un second mode opératoire pour lequel la fréquence est modifiée en continu tandis que la largeur d'impulsions reste constante en pourcentage.

4. Dispositif selon la revendication 1 ou 2, **caractérisé en ce que** l'autre mode opératoire qui existe au moins est un troisième mode opératoire pour lequel la largeur d'impulsions est modifiée en continu tandis que la fréquence reste constante.

5. Dispositif selon l'une des revendications précédentes, **caractérisé en ce que** le rapport de temps entre une courbe dynamique et une courbe statique d'un champ magnétique est réglable librement.

6. Dispositif selon l'une des revendications précédentes, **caractérisé en ce que** des valeurs de seuil pour tous les modes opératoires sont réglables librement.

7. Dispositif selon l'une des revendications précédentes, **caractérisé en ce que** des constantes de temps nécessaires pour les différents modes opératoires sont toutes réglables librement.

8. Dispositif selon l'une des revendications précédentes, **caractérisé en ce que** les modes opératoires ont des courbes de signal avec un nombre de périodes variable.

9. Dispositif selon l'une des revendications précédentes, **caractérisé en ce que** les modes opératoires ont des courbes de signal avec un nombre de périodes constant.

10. Dispositif selon l'une des revendications précédentes, **caractérisé par** un générateur qui alimente au moins une bobine d'inductance pour la production du champ électromagnétique avec du courant alternatif sous forme de fonctions d'oscillation périodiques.

11. Dispositif selon la revendication 10, **caractérisé en ce que** le générateur présente une commande librement programmable avec une interface utilisateur.

12. Dispositif selon la revendication 11, **caractérisé en ce que** la commande librement programmable est constituée sur la base d'un microprocesseur.

13. Procédé pour la commande d'un générateur de fréquence pour la production d'un champ électromagnétique, cependant qu'il produit, à la sortie du générateur de fréquence, des fonctions d'oscillation ou de changement périodiques de formes quelconques en conformité avec un premier mode opératoire et pour modifier celles-ci en continu en conformité avec au moins un autre mode opératoire, **caractérisé en ce que** l'autre mode opératoire qui existe au moins est un mode opératoire combiné pour lequel aussi bien une fréquence qu'une largeur d'impulsions sont modifiées en continu de telle manière que le flux magnétique reste constant.

**Fig. 1**

a)

b)

**Fig. 2**

**Fig. 3**

Fig. 4

Fig. 5

**Fig. 6**

**Fig. 7**

Fig. 8

Fig. 9

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2003028071 A1